# FASCICULE DE BREVET EUROPEEN

(11) **EP 0 998 313 B1**
(45) Date de publication et mention de la délivrance du brevet: **22.08.2001**
(21) Numéro de dépôt: 98940315.9
(22) Date de dépôt: 22.07.1998
(51) Int. Cl.: A61L 31/00

(54) **PROTHESE COMPOSITE POUR LA PREVENTION DES ADHERENCES POST-CHIRURGICALES ET SON PROCEDE D'OBTENTION**
KOMPOSIT-PROTHESE ZUR VERHINDERUNG POSTCHIRURGISCHER VERKLEBUNG UND VERFAHREN ZU IHRER HERSTELLUNG
COMPOSITE PROSTHESIS FOR PREVENTING POST-SURGICAL ADHESIONS AND METHOD FOR OBTAINING SAME

(30) Priorité: 01.08.1997 FR 9710102
(43) Date de publication de la demande: 10.05.2000
(73) Titulaire: SOFRADIM PRODUCTION, 01600 Trévoux (FR)
(72) Inventeur: ORY, François, Régis, F-69270 Fontaines-Saint-Martin (FR); THERIN, Michel, F-69002 Lyon (FR)
(74) Mandataire: Guerre, Dominique
(86) Numéro de dépôt international: FR9801624
(87) Numéro de publication internationale: WO9906079

(56) Documents cités:
- EP-A- 0 372 969
- WO-A-89/02445
- WO-A-93/11805
- WO-A-95/18638
- WO-A-96/08277

## Description

La présente invention concerne une prothèse composite pour la prévention des adhérences post-chirurgicales, notamment dans le domaine de la chirurgie viscérale, pariétale ou neurologique. L'invention sera plus particulièrement décrite par rapport à une prothèse composite destinée à une utilisation en chirurgie pariétale, dans la réparation des éventrations, ou hernies.

Les adhérences post-chirurgicales comprennent toutes les liaisons fibreuses non anatomiques, induites fortuitement par un acte chirurgical lors du processus normal de cicatrisation. Elles peuvent survenir dans toutes les disciplines chirurgicales quel que soit le geste considéré. Elles sont généralement d'autant plus sévères que le traumatisme chirurgical est important et que les tissus assurant normalement les plans de clivage (tissu conjonctif interstitiel, les synoviales, les gaines tendineuses, séreuses péritonéale et pleurale...) ont été touchés. Tout traumatisme chirurgical tissulaire est suivi d'une cascade d'événements physiologiques dont les principaux temps peuvent être simplifiés comme suit :
- temps zéro (t0) : traumatisme chirurgical, effraction capillaire ;
- temps zéro plus quelques minutes : coagulation, formation du réseau fibrineux, libération des facteurs chimiotactiques ;
- temps zéro (t0) plus 12 à 48 heures : afflux leucocytaire à dominante polynucléaire ;
- temps zéro (t0) plus 24 heures à 5 jours : afflux leucocytaire à dominante macrophagique ;
- temps zéro (t0) plus 4 à 8 jours : afflux fibroblastique ;
- temps zéro (t0) plus 5 à 14 jours : différenciation conjonctive de la réaction cicatricielle ;
- temps zéro (t0) plus 15 à 180 jours : remodelage cicatriciel.

Même si les mécanismes exacts sont pour certains encore inconnus, notamment en ce qui concerne le déterminisme de l'intensité de la réaction, il apparaît donc que les premiers jours sont déterminants puisqu'ils conditionnent l'afflux fibroblastique responsable de la formation d'adhérences.

De ce fait, de telles adhérences post-chirurgicales peuvent provoquer des syndromes pouvant se classer principalement en douleurs chroniques, syndromes occlusifs, et infertilité féminine. Par ailleurs, elles augmentent très sensiblement les risques de fausses routes lors d'une réintervention, (effraction myocardique ou intestinale lors de la thoracotomie ou laparotomie itérative), tout en prolongeant les temps opératoires, la dissection préalable pouvant être dans de tels cas très fastidieuse.

Une solution à ce problème consiste en l'interposition d'une barrière physique entre les structures que l'on souhaite ne pas voir adhérer. L'effet barrière recherché pose toutefois le problème du pouvoir adhésiogène intrinsèque de cette barrière. En effet, si la barrière est constituée d'un matériau non résorbable, elle peut être à l'origine elle-même d'adhérences au cours du temps ; et si elle est résorbable, sa résorption doit être suffisamment peu inflammatoire pour ne pas engendrer elle-même des adhérences.

Plusieurs propriétés sont donc nécessaires pour qu'un matériau puisse prétendre réduire le risque d'adhérences, à savoir, entre autres :
- le matériau doit être sensiblement lisse et non poreux, sur au moins l'une de ses faces, de façon à ne pas offrir d'espace à une recolonisation cellulaire ;
- la surface du matériau doit limiter l'adhésion cellulaire primitive.

Toutefois, et notamment dans la chirurgie viscérale et pariétale, la barrière doit également présenter une certaine résistance mécanique lui permettant de remplir sa fonction en tant qu'élément de reconstruction chirurgicale. De manière générale, les tissus prothétiques connus notamment dans le traitement des insuffisances pariétales, par exemple hernies et éventrations, apportent un complément de résistance mécanique à la reconstruction chirurgicale. De tels tissus sont d'autant plus efficaces et leur tolérance locale d'autant meilleure que leur intégration tissulaire est intime et précoce. Pour cette raison les tissus prothétiques connus les plus performants dans ces indications sont généralement très poreux, et conçus de façon à être intégrés dans le corps le plus rapidement possible. Par "poreux", on entend la caractéristique selon laquelle au moins l'une des faces du tissu est rugueuse, pour présenter des alvéoles, réparties régulièrement ou non, et favorisant toute colonisation cellulaire. C'est ainsi que lors du contact avec les viscères par exemple, ces tissus sont adhésiogènes, ce qui limite leur utilisation aux sites dits pré- ou rétropéritonéaux. or, dans un certain nombre de cas, et plus particulièrement lors d'éventrations multirécidivées, l'implantation en site prépéritonéal strict est difficile, voire impossible du fait de l'existence d'un déficit en séreuse étendu.

Un besoin s'est donc fait ressentir de disposer d'un produit permettant de résoudre le problème de prévention des adhérences post-chirurgicales, tout en offrant un renfort prothétique soumis à la recolonisation cellulaire et intégration tissulaire, et pouvant servir, par exemple, à traiter une éventration avec perte péritonéale importante, ou encore des petites éventrations par laparoscopie, et des hernies.

La demande de brevet WO-A-96/08277 décrit à cette fin une prothèse composite comprenant un tissu prothétique, en l'occurrence un treillis résorbable ou non, et au moins un film d'un matériau collagénique réticulé, résorbable, en l'occurrence un gel de collagène coagulé à l'état sec, associé à une face du tissu prothétique. La prothèse composite ainsi constituée trouve application dans le traitement des éventrations et des hernies, et évite, selon les inventeurs, des adhérences postopératoires, car la membrane collagénique constitue une zone de clivage permettant la libération des éventuelles adhérences post-opératoires précoces qui peuvent se former.

La prothèse composite selon le document WO-A-96/08277 doit être améliorée quant à la nécessaire indépendance, une fois qu'elle est implantée, entre, d'un côté, le phénomène de colonisation cellulaire et d'insertion tissulaire, qui doit être si possible dirigé, et de l'autre côté, la résorption du film, qui doit être relativement rapide in vivo, de manière à limiter les phénomènes inflammatoires.

Tel est l'objet de la présente invention.

Conformément à l'invention, en coopération, d'une part le tissu prothétique a une structure tridimensionnelle séparant ses deux faces, dont au moins l'une est ouverte à toute colonisation cellulaire post-chirurgicale, et d'autre part le film du matériau résorbable est lié au moins superficiellement à l'autre face dudit tissu.

Par "face ouverte", on entend que ladite face comporte des alvéoles ayant une certaine profondeur selon l'épaisseur du tissu tridimensionnel, ces alvéoles traversant complètement, ou non, l'épaisseur du tissu, d'une face à l'autre. Dans le cas d'une traversée complète des alvéoles, on parlera d'un tissu prothétique ajouré ou ayant une structure ajourée.

Selon l'invention, préférentiellement la face du film résorbable, opposée au tissu prothétique est sensiblement lisse et non poreuse.

Préférentiellement, le film résorbable est constitué par au moins un dérivé de polysaccharide formant un hydrogel insoluble en milieu aqueux.

Grâce à l'invention, de manière contrôlable :
- la prothèse empêche la colonisation cellulaire immédiate post-chirurgicale, du côté comportant le film résorbable, lequel se résorbe pendant une durée compatible avec la reconstitution tissulaire, par exemple celle du péritoine ;
- la prothèse facilite la colonisation cellulaire immédiate post-chirurgicale, sur la face comportant le tissu, ouverte de manière à permettre une intégration rapide et mécaniquement efficace de celle-ci, notamment lorsqu'elle est utilisée en tant que renfort pariétal ou viscéral.

Préférentiellement, mais de manière non exclusive, le tissu prothétique comprend deux faces poreuses opposées, reliées l'une à l'autre par des fils de liaison, dont l'une est ouverte à toute colonisation cellulaire post-chirurgicale, et dont l'autre est fermée à ladite colonisation par le film de matériau résorbable. Par exemple, l'armure du tissu prothétique détermine dans l'épaisseur de ce dernier, une multiplicité d'alvéoles ou canaux transversaux, sensiblement parallèles les uns aux autres, débouchant de part et d'autre dudit tissu sur les deux faces poreuses respectivement, et dont la section interne est substantiellement exempte de tout fil de liaison. Il s'agit donc d'un tissu prothétique souple ayant une structure en "nid d'abeille".

Par dérivé de polysaccharide, on entend aussi bien le polysaccharide considéré à l'état pur, que ce dernier modifié chimiquement, ou mélangé à d'autres produits ou adjuvants biocompatibles.

Avantageusement, le polysaccharide est choisi dans le groupe consistant en les mucopolysaccharides, les polysaccharides polyanioniques, les glycosaminoglycannes, les celluloses modifiées, et des mélanges de ceux-ci. De préférence, le dérivé de polysaccharide est choisi dans le groupe consistant en un dérivé de l'acide hyaluronique (HA) ou de ses sels, un dérivé de la carboxyméthylcellulose (CMC), un dérivé de la carboxyméthylamylose (CMA), un dérivé du chondroïtine-6-sulfate, un dérivé du dermatine sulfate, un dérivé de l'héparine et un dérivé de l'héparine sulfate, ou un mélange de ceux-ci.

Plus préférentiellement encore, le dérivé de polysaccharide est un dérivé de l'acide hyaluronique ou l'un de ses sels hyaluroniques. L'acide hyaluronique est un mucopolysaccharide naturel présent, entre autres, dans le fluide synovial, dans les parois des vaisseaux sanguins, le cordon ombilical et dans d'autres tissus conjonctifs. Le polysaccharide consiste en des résidus de N-acétyl-D-glucosamine et d'acide D-glucuronique, reliés par des liaisons β 1-3 glucuronidiques et β 1-4 glucosaminidiques, respectivement, de sorte que l'unité de construction est désignée -(1>4)-β-D-GlcA-(1>3)-β-D-GlcNAc. Le HA se dissout dans l'eau et forme un liquide à haute viscosité. Le poids moléculaire de HA isolé de sources naturelles est généralement compris entre 5x10⁴ à 1x10⁷ Daltons. Tel qu'utilisé dans la présente demande, le terme "HA" comprend aussi bien l'acide hyaluronique, que ses sels hyaluroniques, et inclue par exemple, l'hyaluronate de sodium, l'hyaluronate de potassium, l'hyaluronate de magnésium, et l'hyaluronate de calcium.

Le dérivé de HA préféré est insoluble dans l'eau, et biocompatible, et peut être obtenu, par exemple en faisant réagir le HA avec un agent réticulant polyfonctionnel, tel que par exemple un époxyde polyfonctionnel. De manière générale, les procédés de réticulation ou de modification permettant d'obtenir un dérivé de HA convenable pour une association au tissu prothétique tridimensionnel sont bien connus en soi et ne seront pas décrits plus en détails ici. Ces procédés sont notamment décrits dans les demandes de brevet WO-A-89/02445, WO-A-92/00105, et WO-A-92/20349.

Avantageusement, l'épaisseur du film résorbable est moins importante que l'épaisseur du tissu prothétique, par exemple comprise entre 2% et 10% de l'épaisseur totale de la prothèse composite, et de préférence comprise entre environ 30 *µ*m et 100 *µ*m, et plus préférentiellement est d'environ 50 *µ* m à 75 *µ*m.

Le film résorbable qui fait partie de la prothèse composite de l'invention est biocompatible, non toxique, et se résorbe in vivo rapidement. Le matériau résorbable utilisé peut être indifféremment d'origine animale, humaine ou synthétique.

Dans un mode d'exécution préféré, le film résorbable est lié au moins superficiellement au tissu prothétique, directement ou indirectement, et de préférence est lié directement par absorption capillaire, dans une certaine épaisseur du matériau, dans les fibres constitutives du tissu prothétique.

Plus préférentiellement encore, le film résorbable est lié directement par absorption capillaire du matériau résorbable dans les fibres constitutives du tissu prothétique sur une profondeur inférieure à 750 *µ*m, mesurée à partir la surface extérieure du film.

Conformément à l'invention, une prothèse composite comporte deux faces différentes dans leurs aspects et fonctions respectifs, à savoir une face poreuse ou ouverte d'un côté, pour accueillir et diriger la colonisation cellulaire post-chirurgicale, et l'autre face fermée pour la séparation tissulaire sans adhérence.

Le film résorbable est de préférence continu, lisse et non poreux, recouvrant entièrement le tissu prothétique, et plus préférentiellement, déborde de ce dernier de façon à protéger la prothèse de contacts viscéraux, le débord pouvant être par exemple de 5 à 10 millimètres.

Le film résorbable est intimement lié au tissu par pénétration superficielle, de façon à ne pas constituer un plan de clivage ou délamination, tout en maintenant la porosité du tissu ouverte sur l'autre face.

De préférence, le film résorbable est également souple, en particulier à l'état hydraté, de façon à préserver la maniabilité de la prothèse, et son éventuelle utilisation par voie coelioscopique.

Une fois réhydraté, le film restaure au tissu prothétique ses propriétés mécaniques initiales (souplesse et élasticité) sans se fragmenter, ni rendre la fixation de la prothèse plus difficile. Il est en outre transparent, non délaminable et non collant lors de sa mise en place. Sa résorption rapide assure la protection contre les phénomènes adhésiogènes initiaux, c'est-à-dire dans la première semaine post-opératoire, ou autrement dit, pendant le laps de temps nécessaire à l'intégration tissulaire de la face opposée. Lors de sa résorption, son caractère faiblement inflammatoire et/ou immunogène ne perturbe pas la colonisation tissulaire à l'opposé dudit film.

La présente invention sera mieux comprise par la description détaillée d'un mode d'exécution préféré, donnée à titre d'exemple, par référence au dessin en annexe, dans lequel :
- la **Figure 1** représente schématiquement une prothèse composite selon la présente invention ;
- la **Figure 2** représente un dessin schématique de l'armure de tricotage d'un tissu prothétique appartenant à une prothèse composite selon la présente invention.

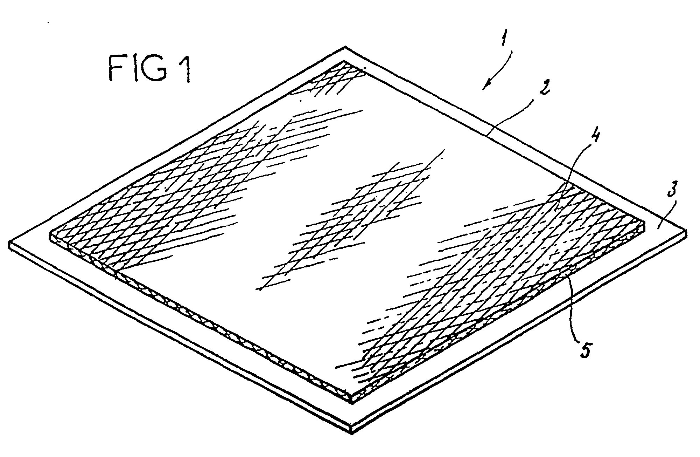

En se référant utilement à la Figure 1, une prothèse composite selon la présente invention est représentée de manière générale par la référence 1. La prothèse comporte un tissu prothétique 2, présentant deux faces 4 et 5, dont l'une est recouverte d'un film 3 de dérivé de polysaccharide. Le tissu prothétique 2 a une structure tridimensionnelle ajourée, et donc une certaine épaisseur qui sépare la face 4 de la face 5 Ce tissu peut être de manière préférée un tricot Rachel® à entretoise, réalisé sur double fonture. L'écartement des deux fontures et les débits des fils permettent d'obtenir un tissu fini en trois dimensions (structure tridimensionnelle), d'une épaisseur comprise entre 1 à 3 mm, et par exemple d'environ 1,8 mm, pour un poids d'environ 90 g/m². Les caractéristiques finales sont données indépendamment du tricotage par le choix de la matière première employée, par exemple du polyester PES 50 dtex multifilaments, la température, et le temps de thermofixage. En dehors de la filature, le fil et le tissu ne reçoivent aucun autre traitement (pas d'ensimage ou de lavage). Un tel tissu présente, selon la norme NFG 07119, une force de rupture par traction comprise entre environ 18 daN et environ 30 daN, et un allongement de rupture par traction compris entre environ 25% à 37%, en chaîne, et une force de rupture par traction comprise entre environ 9 daN et 15 daN, et un allongement de rupture par traction compris entre 60% à 88%, en trame.

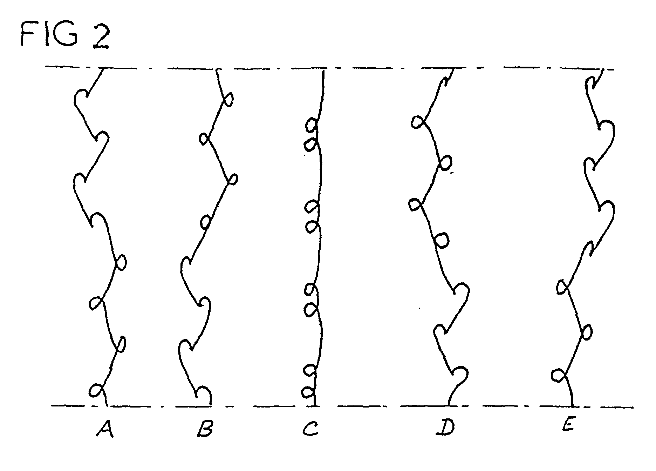

Un tel tissu composite peut être réalisé par tricotage en chaîne de cinq nappes de fils, et conformément au dessin schématique de la Figure 2. Dans cette figure, chaque nappe de fils est identifiée par une lettre, allant de A à E, le schéma en lui-même utilisant un système de description du tricot à réaliser tout à fait habituel et compréhensible pour l'homme du métier, et qui ne sera pas décrit plus en détails ici. Conformément à la Figure 2, le tissu prothétique préféré selon la présente invention est constitué, comme précédemment décrit, de deux faces poreuses indépendantes. Ces deux faces sont, dans l'exemple donné, elles-mêmes constituées de deux nappes de fils, référencées A,B et D,E respectivement, les nappes A,B donnant une face à ouvertures en forme de goutte d'eau, pour accueillir et diriger la colonisation cellulaire post-chirurgicale, et les nappes D,E donnant une face à ouvertures hexagonales qui sera fermée par le film du matériau collagénique. Le tissu prothétique peut être tricoté sur un métier Rachel à double fonture. Dans ce cas, toutes les barres correspondant aux fils A,B et D,E sont enfilées un plein-un vide. La nappe de fils de liaison est représentée par la référence C, et est enfilée pleine. Les différentes nappes A-E de fils sont toutes tricotées en même temps. Ainsi, les fils de liaison sont distribués selon les bords périphériques des ouvertures de chaque face et s'étendent sensiblement perpendiculairement depuis une face vers l'autre face, évitant que des fils de liaison occupent une partie trop importante du volume des canaux transversaux qui sont formés. Le tissu final peut ensuite être stabilisé simplement par passage au four à une température comprise entre environ 170°C et environ 220°C.

Il résulte de la description précédente que les fils constitutifs du tissu tridimensionnel ont une nature non résorbable, mais biocompatible, par conséquent différente de celle du matériau résorbable du film.

La fabrication d'une prothèse composite associant un tissu prothétique à structure ajourée tridimensionnelle, tel qu'obtenu précédemment, avec un film de dérivé de polysaccharide peut être réalisée comme suit.

La solution contenant le dérivé de polysaccharide est répartie uniformément sur un support inerte hydrophobe et plan pour former un film résultant de deux couches minces superposées. Pour ce faire, on applique d'abord une première couche mince de la solution. Après gélification de cette première couche mince par refroidissement, on applique à sa surface une deuxième couche mince à partir de la même solution.

Le tissu prothétique à structure ajourée tridimensionnelle, présentant une épaisseur de l'ordre de 1,8 mm, est appliqué par sa face à ouvertures hexagonales sur la deuxième couche mince, avant gélification, de telle sorte que l'ancrage du tissu dans le dérivé de polysaccharide s'effectue pendant le séchage du film. Après réaction, la prothèse composite est séparée du support inerte hydrophobe.

Il est remarqué que le film de dérivé de polysaccharide peut remonter par capillarité dans les fibres, cet effet étant en partie responsable de la résistance à la délamination élevée du film du tissu prothétique. Enfin, le film est continu, aucune fibre synthétique provenant du tissu prothétique n'apparaissant à sa surface. Par ailleurs, le film présente une épaisseur de l'ordre de 50 *µ*m à 75*µ*m, mais peut remonter par capillarité dans les fibres du tissu prothétique jusqu'à une épaisseur d'environ 750 *µ*m.

## Revendications

1. Prothèse composite, comprenant un tissu prothétique non résorbable et un film d'un matériau résorbable in vivo, associé à une face du tissu prothétique, caractérisée en ce que, le tissu prothétique a une structure tridimensionnelle séparant deux faces poreuses opposées et reliées l'une à l'autre par des fils de liaison, l'une desdites faces poreuses étant ouverte à toute colonisation cellulaire post-chirurgicale, et l'autre desdites faces poreuses étant fermée à ladite colonisation par le film de matériau résorbable, ledit film étant lié, superficiellement, mais directement par absorption capillaire d'au moins un dérivé de polysaccharide formant un hydrogel insoluble en milieu aqueux dans les fibres constitutives du tissu prothétique.

2. Prothèse selon la revendication 1, caractérisée en ce que l'armure du tissu prothétique détermine dans l'épaisseur de ce dernier, une multiplicité d'alvéoles ou canaux transversaux, sensiblement parallèles les uns aux autres, débouchant de part et d'autre dudit tissu sur les deux faces poreuses respectivement, et dont la section interne est substantiellement exempte de tout fil de liaison.

3. Prothèse selon la revendication 2, caractérisée en ce que le tissu prothétique présente une structure dans laquelle chaque canal ou alvéole transversal a une paroi poreuse d'interconnexion avec les canaux voisins, telle qu'obtenue par tricotage en chaîne, sur un métier RACHEL® à double fonture, d'au moins cinq nappes de fils, à savoir quatre nappes A, B, D et E, constituant deux à deux les deux faces respectivement et au moins une nappe (C) de fils de liaison, les barres correspondant aux fils (A, B, D, E) desdites quatre nappes respectivement étant enfilées un plein-un vide.

4. Prothèse composite selon la revendication 3, caractérisée en ce que le dérivé de polysaccharide est choisi dans le groupe consistant en les mucopolysaccharides, les polysaccharides polyanioniques, les glycosaminoglycanes, les celluloses modifiées, et des mélanges de ceux-ci.

5. Prothèse composite selon la revendication 3, caractérisée en ce que le dérivé de polysaccharide est choisi dans le groupe consistant en un dérivé de l'acide hyaluronique (HA) ou l'un de ses sels hyaluroniques, un dérivé de la carboxyméthylcellulose (CMC), un dérivé de la carboxyméthylamylose (CMA), un dérivé du chondroïtine-6-sulfate, un dérivé du dermatine sulfate, un dérivé de l'héparine et un dérivé de l'héparine sulfate, ou un mélange de ceux-ci.

6. Prothèse composite selon la revendication 3, caractérisée en ce que le dérivé de polysaccharide est un dérivé de l'acide hyaluronique (HA), ou l'un de ses sels hyaluroniques.

7. Prothèse composite selon la revendication 1, caractérisée en ce que l'épaisseur du film résorbable est moins importante que l'épaisseur du tissu prothétique.

8. Prothèse composite selon la revendication 1, caractérisée en ce que l'épaisseur du film résorbable est comprise entre 2% et 10 % de l'épaisseur totale de la prothèse composite.

9. Prothèse composite selon la revendication 1, caractérisée en ce que l'épaisseur du film résorbable est comprise entre environ 30 *µ*m à 100 *µ* m, et de préférence est comprise entre environ 50 *µ*m à 75 *µ*m.

10. Prothèse composite selon la revend cation 1, caractérisée en ce que le film est lié aux fibres constitutives du tissu prothétique, sur une profondeur inférieure à 750 *µ*m, mesurée à partir de la surface extérieure du film.

## Patentansprüche

1. Verbundprothese, die ein nicht-resorbierbares Prothesengewebe und einen Film aus einem in vivo resorbierbaren Material aufweist, der mit einer Seite des Prothesengewebes verbunden ist, dadurch gekennzeichnet, daß das Prothesengewebe eine dreidimensionale Struktur aufweist, die zwei sich gegenüberliegende und durch Verbindungsfäden miteinander verbundene poröse Seiten voneinander trennt, wobei die eine der porösen Seiten für jegliche post-chirurgische zelluläre Kolonisierung zugänglich ist, und die andere der porösen Seiten durch den Film aus resorbierbarem Material für die Kolonisierung unzugänglich ist, wobei der Film oberflächlich, aber direkt durch kapillare Absorption von zumindest einem Polysaccharidderivat, das ein in wäßrigem Milieu unlösliches Hydrogel bildet, in den das Prothesengewebes bildenden Fasern gebunden ist.

2. Prothese nach Anspruch 1, dadurch gekennzeichnet, daß die Bindung des Prothesengewebes in dessen Dicke eine Vielzahl von Alveolen oder im wesentlichen parallel zueinander verlaufende durchquerende Kanäle bestimmt, die zu beiden porösen Seiten des Gewebes münden, und deren Innenquerschnitt im wesentlichen frei von jeglichem Verbindungsfaden ist.

3. Prothese nach Anspruch 2, dadurch gekennzeichnet, daß das Prothesengewebe eine Struktur aufweist, in der jeder durchquerende Kanal oder Alveole mit den benachbarten Kadurchquerende Kanal oder jede Alveole mit den benachbarten Kanälen eine poröse Verbindungswand aufweist, wie sie beispielsweise durch Kettstricken von zumindest fünf Fadenscharen auf einer RACHEL®-Doppelfontur-Strickmaschine erhalten wird, und zwar von vier Scharen A, B, D und E, die jeweils paarweise die beiden Seiten bilden, und von zumindest einer Schar (C) von Verbindungsfäden, wobei die Barren, die den Fäden (A, B, D, E) der vier Scharen entsprechen, 'eines voll-eines leer' eingefädelt werden.

4. Verbundprothese nach Anspruch 3, dadurch gekennzeichnet, daß das Polysaccharidderivat aus der Gruppe ausgewählt ist, die aus den Mucopolysacchariden, den polyanionischen Polysacchariden, den Glycosaminglykanen, den modifizierten Cellulosen und Gemischen daraus besteht.

5. Verbundprothese nach Anspruch 3, dadurch- gekennzeichnet, daß das Polysaccharidderivat aus der Gruppe ausgewählt ist, die aus einem Hyaluronsäure(HA)-Derivat oder einem ihrer Hyaluronsalze, einem Carboxymethylcellulose(CMC)-Derivat, einem Carboxymethylamylose(CMA)-Derivat, einem Chondroitin-6-Sulfat-Derivat, einem Dermatansulfatderivat, einem Heparinderivat und einem Heparinsulfatderivat, oder einem Gemisch daraus besteht.

6. Verbundprothese nach Anspruch 3, dadurch gekennzeichnet, daß das Polysaccharidderivat ein Hyaluronsäure(HA)-Derivat oder eines ihrer Hyaluronsalze ist.

7. Verbundprothese nach Anspruch 1, dadurch gekennzeichnet, daß die Dicke des resorbierbaren Films kleiner ist als die Dicke des Prothesengewebes.

8. Verbundprothese nach Anspruch 1, dadurch gekennzeichnet, daß die Dicke des resorbierbaren Films im Bereich zwischen 2 % und 10 % der Gesamtdicke der Verbundprothese liegt.

9. Verbundprothese nach Anspruch 1, dadurch gekennzeichnet, daß die Dicke des resorbierbaren Films im Bereich zwischen etwa 30 *µ*m und 100 *µ*m liegt, und vorzugsweise zwischen etwa 50 *µ*m und 75 *µ* m liegt.

10. Verbundprothese nach Anspruch 1, dadurch gekennzeichnet, daß der Film an die das Prothesengewebe bildenden Fasern auf einer Tiefe kleiner als 750 *µ*m, gemessen von der äußeren Oberfläche des Films aus, gebunden ist.

## Claims

1. Composite prosthesis, comprising a non-absorbable prosthetic fabric and a film made of a material which is absorbable in vivo, combined with one surface of the prosthetic fabric, characterized in that the prosthetic fabric has a three-dimensional structure separating two opposite porous surfaces and which are connected to one another by connecting yarns, one of said porous surfaces being open to all post-surgical cell colonization, and the other of said porous surfaces being closed to said colonization by means of the film of absorbable material, said film being superficially, but directly linked by capillary absorption of at least one polysaccharide derivative forming a hydrogel which is insoluble in aqueous medium in the constituent fibers of the prosthetic fabric.

2. Prosthesis according to Claim 1, characterized in that the weave of the prosthetic fabric determines, within the thickness of the latter, a multiplicity of alveoli or transverse channels, substantially parallel to one another, opening out on either side of said fabric on the two porous surfaces respectively, and of which the internal section is substantially free of any connecting yarn.

3. Prosthesis according to Claim 2, characterized in that the prosthetic fabric has a structure in which each transverse channel or alveolus has a porous wall interconnecting with the adjacent channels, for example obtained by warp knitting, on a double-rib RACHEL® loom, of at least five layers of yarn, namely four layers A, B, D and E, constituting in pairs the two surfaces, respectively, and at least one layer (C) of connecting yarns, the bars corresponding to the yarns (A, B, D, E) of said four layers respectively being threaded one full/one empty.

4. Composite prosthesis according to Claim 3, characterized in that the polysaccharide derivative is chosen from the group consisting of mucopolysaccharides, polyanionic polysaccharides, glycosaminoglycans, modified celluloses, and mixtures of these.

5. Composite prosthesis according to Claim 3, characterized in that the polysaccharide derivative is chosen from the group consisting of a derivative of hyaluronic acid (HA) or one of its hyaluronic salts, a derivative of carboxymethylcellulose (CMC), a derivative of carboxymethylamylose (CMA), a derivative of chondroitin-6-sulfate, a derivative of dermatan sulfate, a derivative of heparin and a derivative of heparin sulfate, or a mixture of these.

6. Composite prosthesis according to Claim 3, characterized in that the polysaccharide derivative is a derivative of hyaluronic acid (HA), or one of its hyaluronic salts.

7. Composite prosthesis according to Claim 1, characterized in that the thickness of the absorbable film is less than the thickness of the prosthetic fabric.

8. Composite prosthesis according to Claim 1, characterized in that the thickness of the absorbable film is between 2% and 10% of the total thickness of the composite prosthesis.

9. Composite prosthesis according to Claim 1, characterized in that the thickness of the absorbable film is between about 30 µm to [sic] 100 µm, and preferably between about 50 µm to [sic] 75 µm.

10. Composite prosthesis according to Claim 1, characterized in that the film is linked to the constituent fibers of the prosthetic fabric, over a depth of less than 750 µm, measured from the outer surface of the film.
